# EUROPEAN PATENT APPLICATION

(11) **EP 2 860 526 A1**
(43) Date of publication of application: **15.04.2015**
(21) Application number: 13306389.1
(22) Date of filing: 08.10.2013
(51) Int. Cl.: G01N 33/68

(54) **Methods for the diagnosis and/or the prognosis of Myotonic Dystrophy type 1 (DM1)**

(71) Applicant: INSTITUT NATIONAL DE LA SANTE ET DE LA RECHERCHE MEDICALE (INSERM), 75013 Paris (FR)
(72) Inventor: The designation of the inventor has not yet been filed
(74) Representative: Nony

(57) **Abstract**

An *in vitro* method for detecting the occurrence of Myotonic Dystrophy type 1 (DM1) disease in an individual, comprising the steps of:
a) providing a blood sample from said individual,
b) measuring an amount of Aβ peptide within said blood sample,
c) comparing the amount measured at step b) with a reference value,
d) determining the occurrence of DM1 in said individual from the comparison performed at step c).

SEQUENCE LISTING

<110> INSERM

<120> Biomarker DM1

<130> 98716

<160> 5

<170> BiSSAP 1.0

<210> 1
<211> 770
<212> PRT
<213> Homo sapiens

<220>
<221> SOURCE
<222> 1..770
<223> /mol_type="protein"
      /organism="Homo sapiens"

<400> 1

<210> 2
<211> 40
<212> PRT

<213> Homo sapiens

<220>
<221> SOURCE
<222> 1..40
<223> /mol_type="protein"
      /organism="Homo sapiens"

<400> 2
Asp Ala Glu Phe Arg His Asp Ser Gly Tyr Glu Val His His Gln Lys
1               5                   10                  15
Leu Val Phe Phe Ala Glu Asp Val Gly Ser Asn Lys Gly Ala Ile Ile
            20                  25                  30
Gly Leu Met Val Gly Gly Val Val
        35                  40

<210> 3
<211> 42
<212> PRT
<213> Homo sapiens

<220>
<221> SOURCE
<222> 1..42
<223> /mol_type="protein"
      /organism="Homo sapiens"

<400> 3

<210> 4
<211> 23
<212> DNA
<213> Homo sapiens

<220>
<221> source
<222> 1..23
<223> /mol_type="DNA"
      /note="Primer"
      /organism="Homo sapiens"

<400> 4
cacaggctga agtggcagtt cca                                              23


<210> 5
<211> 22
<212> DNA
<213> Homo sapiens

<220>
<221> source
<222> 1..22
<223> /mol_type="DNA"
      /note="Primer "
      /organism="Homo sapiens"

<400> 5
tgtcggggtc tcagtgcatc ca                                                22

## Description

### FIELD OF THE INVENTION

The invention relates to methods for the diagnosis and/or the prognosis of Myotonic Dystrophy type 1 (DM1) disease from the blood sample of an individual, more particularly for detecting the occurrence of brain dysfunction in individuals suffering from DM1. The invention further relates to methods for the screening of compounds potentially useful in the treatment of DM1 in said individual.

### BACKGROUND OF THE INVENTION

Myotonic dystrophy type I (DM1) is a dominantly inherited multisystemic neuromuscular disorder. Fronto-temporal cognitive impairment is often reported in adult DM1 affected patients; however the identification of biomarkers of brain involvement in DM1 is still lacking.

Recently, a reduced concentration of Aβ₁₋₄₂ in the cerebrospinal fluid (CSF) of patients with DM1 has been described in adult DM1 (Winblad *et al.,* 2008) and in juvenile DM1, which is defined by an age of onset below 20 years old (Peric *et al.,* 2013).

However, the above mentioned methods are not fully satisfactory, because they rely necessarily on the measurement of Aβ peptides in the cerebrospinal fluid (CSF), which is a particularly invasive procedure with uncertain benefit, and which is not well suited for routine and/or daily medical practice.

Thus, there remains a need for alternative, more simple, methods for detecting the occurrence of brain dysfunction in an individual with Myotonic Dystrophy type 1 (DM1) disease.

There also remains a need for alternative methods for discriminating between DM1 and a non-DM1 neurodegenerative disease in an individual.

In particular, there remains a need for methods which are more acceptable and less painful for the patient, and which may be followed in place of, or in complement of other diagnostic or prognosis methods.

In view of the above, there also remains a need for novel methods for determining the efficacy of a treatment of Myotonic Dystrophy type 1 (DM1) disease in an individual, as well as an assay for the screening of candidate agents potentially useful in the treatment of DM1.

### SUMMARY OF THE INVENTION

The present invention has for purpose to meet these aforementioned needs.

As shown in the following examples, the concentration of several Aβ species has been measured in the plasma of individuals in order to determine whether plasma Aβ levels or ratios between species of Aβ peptides are modified in DM1 when compared to controls.

Unexpectedly, the inventors have found that plasma Aβ concentrations of several Aβ species in DM1 patients were significantly higher than plasma Aβ concentrations in controls independently of the age or sex of the individuals, and that this variation could be used as a biomarker for the diagnosis and follow-up of patients suffering from DM1.

This result was completely unexpected for at least three reasons: (i) because to the applicant's knowledge, it is described for the first time herein the investigation of a variation of the level of Aβ peptides in a blood sample from individuals with DM1, (ii) because neither amyloid deposits nor a defective metabolism of APP - including Aβ production and clearance - have been previously reported in DM1, and also (iii) because two recent studies have investigated a variation of Aβ peptides (Winblad *et al.,* 2008; Peric et al., 2013) in the CSF, and both had observed a decrease of the level of Aβ₁₋₄₂ in body fluids like CSF in correlation with the disease.

Clearly, the observation in DM1 patients that, on one hand, (i) levels of Aβ peptides in CSF would decrease and that, on the other hand, (ii) levels of Aβ peptides in blood samples would increase over controls could not be anticipated by the one skilled in the art since a recent study also demonstrate no correlation between CSF and plasma Aβ concentrations (Huang et al., 2012).

Thus, a first aspect of the invention is to provide an *in vitro* method for detecting the occurrence of Myotonic Dystrophy type 1 (DM1) disease in an individual, and more particularly the occurrence of brain dysfunction in an individual with Myotonic Dystrophy type 1 (DM1) disease, comprising the steps of:
a) providing a blood sample from said individual,
b) measuring an amount of Aβ peptide within said blood sample,
c) comparing the amount measured at step b) with a reference value,
d) determining the occurrence of DM1 in said individual from the comparison performed at step c).

According to another aspect, the invention relates to an *in vitro* method for discriminating between DM1 and a non-DM1 neurodegenerative disease in an individual, comprising the steps of:
a) providing a blood sample from said individual,
b) measuring an amount of Aβ peptide within said blood sample,
c) comparing the amount of Aβ peptide within said blood sample with a reference value,
d) discriminating between Myotonic Dystrophy type 1 (DM1) disease and a non-DM1 neurodegenerative disease from the comparison performed at step c).

According to a third aspect, the invention relates to an *in vitro* method for determining the efficacy of a treatment of Myotonic Dystrophy type 1 (DM1) disease in an individual, comprising the steps of:
a) providing a blood sample from said individual,
b) measuring an amount of Aβ peptide within said blood sample,
c) comparing the amount measured at step b) with a reference value,
d) determining the efficacy of said treatment in said individual from the comparison performed at step c).

According to a fourth aspect, the invention relates to an assay for the screening of a candidate agent potentially useful in the treatment of DM1 in an individual, said assay comprising determining whether a tested agent can modulate, such as increase or reduce and preferably reduce, the level of an Aβ peptide in a blood sample from said individual.

### BRIEF DESCRIPTION OF THE FIGURES

**Figure 1****:** ROC curve for sensitivity and specificity of the Aβ₁₋₄₂ dosage. (1-Specificity) or False Positive Rate *(x-axis),* and Sensitivity or True Positive Rate *(y-axis),* are indicated according to a probability scale from 0 to 1. Area Under the Curve = 0.6415.
**Figure 2****:** ROC curve for sensitivity and specificity of the Aβ₁₋₄₀ dosage. (1-Specificity) or False Positive Rate *(x-axis),* and Sensitivity or True Positive Rate *(y-axis),* are indicated according to a probability scale from 0 to 1. Area Under the Curve = 0.6622.
**Figure 3****:** ROC curve for sensitivity and specificity of the Aβₓ₋₄₀ dosage. (1-Specificity) or False Positive Rate *(x-axis),* and Sensitivity or True Positive Rate *(y-axis),* are indicated according to a probability scale from 0 to 1. Area Under the Curve = 0.6041.
**Figure 4****:** ROC curve for sensitivity and specificity of the Aβₓ₋₄₂ dosage. (1-Specificity) or False Positive Rate *(x-axis),* and Sensitivity or True Positive Rate *(y-axis),* are indicated according to a probability scale from 0 to 1. Area Under the Curve = 0.6130.

### DETAILED DESCRIPTION OF THE INVENTION

Myotonic dystrophy type I is an autosomal inherited multisystemic neuromuscular disease associated with a dynamically unstable mutation. This mutation consists of a trinucleotide CTG repeat expansion in the 3' untranslated region of the myotonic dystrophy protein kinase (*DMPK*) gene. The brain and cognitive functions are often deteriorated in adult patients and include frontal lobe dysfunction, visuoconstructive disability, anhedonia, difficulties to recognize facial emotion, daytime sleepiness and hypersomnolence (Winblad *et al.,* 2006 ; Meola *et al.* 2007 ; Sistiaga *et al.,* 2010). The *DMPK* gene is ubiquitously expressed including in the brain (Sergeant *et al.,* 2001). Transcription of the CTG repeats produces long CUG stretches that are retained in the cell nucleus and are responsible of an RNA gain-of-toxic function. This toxic gain-of-function principally results in a trans-deregulation of alternative splicing of a growing list of transcripts. Thus, in DM1 brain, the microtubule-associate Tau protein, the N-methyl D-aspartate receptor 1 (GRIN1) or the amyloid precursor protein (APP) splicing are modified leading to re-expression of foetal-type isoforms (Sergeant *et al.,* 2001; Jiang *et al.,* 2004 ; Dhaenens *et al.,* 2008).

Modification of the diversity of protein isoforms may impair the function of those proteins and can be associated with the development of neuropathological lesions. Neurofibrillary tangles characterized by the intraneuronal aggregation of the shortest tau isoform are often affecting the hippocampo-temporo-insular brain regions and subcortical brain nuclei (Vermersch *et al.,* 1996 ; Maurage *et al.,* 2005). Likewise, there is an increased expression of the neuronal APP₆₉₅ isoform and a decrease of non-neuronal APP₇₅₁ and APP₇₇₀ isoforms potentially associated with a modified metabolism of APP (Jiang *et al.,* 2004). Sequential proteolytic cleavages of APP by the β- and γ-secretase generate Aβ peptides of 37 to 43 amino acids. Long forms of Aβ peptides are pro-aggregative and are the main components of parenchymal brain amyloid deposits observed in Alzheimer's disease (AD) (for review see Suh and Checler, 2002). However, amyloid deposits or a defective metabolism of APP have not been reported yet in DM1 (Yoshimura *et al.,* 1990; Kiuchi *et al.,* 1991; Vermersch *et al.,* 1996).

In Alzheimer's Disease (AD), CSF Aβ₁₋₄₂ levels are inversely correlated to the number of brain amyloid deposits and, they may be indicative either of the presence of brain lesions, a defective APP metabolism and / or any change of Aβ clearance. Concentration of Aβ species in CSF is therefore considered as a valuable peripheral central nervous system biomarkers.

So far, neither amyloid deposits nor a defective metabolism of APP have been reported in DM1. However, CSF dosage of Aβ peptides showed lower Aβ₁₋₄₂ concentrations in DM1 patients as compared to controls (Winblad *et al.,* 2008). The magnitude of Aβ₁₋₄₂ reduction was lower than that reported in AD and cannot be associated to amyloid deposition since amyloid deposition is not reported in DM1 brain, whereas it has been reported in AD. Such an Aβ₁₋₄₂ reduction in CSF was measured in juvenile DM1 individuals but not corroborated in the adult cohort (Peric *et al.,* 2013).

In view of the above, it is still a matter of debate whether DM1 should be classified as a neurodegenerative, neurodevelopmental or neurofunctional disease (see Peric *et al.,* 2013), as the mechanism responsible for the CSF reduction of Aβ peptides in DM1 is still under investigation and may differ from other known Aβ-related diseases such as Alzheimer's Disease.

A current hypothesis suggests that the CSF concentration of Aβ peptides may result from changes in the production or clearance of the peptides (Potter *et al.,* 2013). However, no correlation has been found in the prior art between plasma and cerebrospinal fluid (CSF) from healthy individuals, with age, or in individuals with amyloid deposits in the brain (Huang *et al.,* 2012). A positive correlation between Aβ concentration in plasma and age is found but not in CSF (Huang *et al.,* 2012).

Thus, the raise of Aβ concentration in plasma could neither be deduced from the physiopathology of DM1 nor from studies related to Aβ CSF concentrations.

Thus, the inventors now show herein that the amount of Aβ peptide can be assessed from blood samples of individuals suffering from DM1, and that said levels are higher in blood samples from DM1 patients than in blood samples of non-DM1 patients.

Interestingly, this variation in Aβ plasma concentration is independent of the age of the individuals and still significant when corrected for age and sex. Moreover, those results have been found to be significant for distinct Aβ species.

Without wishing to be bound by any particular theory, the inventors cannot exclude that Aβ peptides originate from the brain, and that the reduced concentration of CSF Aβ peptides in DM1 would result from an increased clearance through the blood brain barrier thus potentially modifying blood Aβ concentrations in DM1 patients.

Indeed, Aβ peptides are degraded in the brain parenchyma or cleared through a retrograde transport from the brain to the blood across the blood brain barrier (Spies *et al.,* 2012). Recent studies suggest that apolipoprotein E may contribute to the clearance of Aβ (Arold *et al.,* 2012). Elevated level of ApoE is associated with elevated Aβ in cortical synapses in Alzheimer's disease patients. Alternatively, Aβ clearance is also mediated through the selective permeability of the blood brain barrier and likely involves a soluble form of the lipoprotein-receptor 1 (sLRP1) or the advance glycation end product receptor (RAGE). Itoh and collaborators (2010) have reported hyalinized vessels and perivascular dilation, which could suggest a potential change in the blood brain barrier (BBB) permeability and could contribute to the increased concentration of Aβ in the blood. Change in the perivascular structure and consequently, the blood brain barrier could also result from modification of extracellular matrix protein expression. Splicing modification of several extracellular matrix protein have been reported in DM1 (Du *et al.,* 2010), which together with the neuropathological observations of brain vessels abnormality could contribute to a modified permeability of the blood brain barrier and the leakage of central nervous system polypeptides such as Aβ.

Although speculative, a change in the BBB permeability would not be selective toward Aβ species and thus, several peptides concentration should increase including for instance, microtubule-associated Tau protein, α-synuclein or GFAP. Such an elevation of Aβ species concentration measured in the plasma of DM1 patients without modification of the ratio between Aβ species is observed and therefore supports this hypothesis.

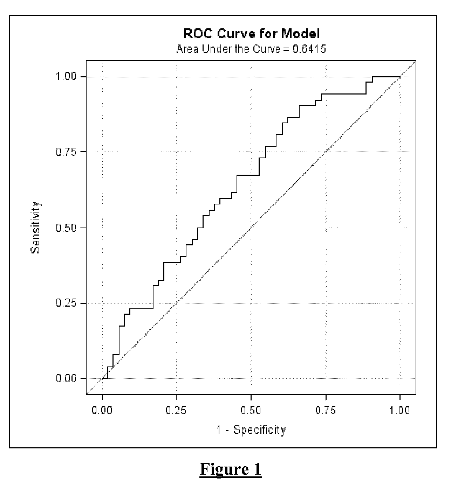

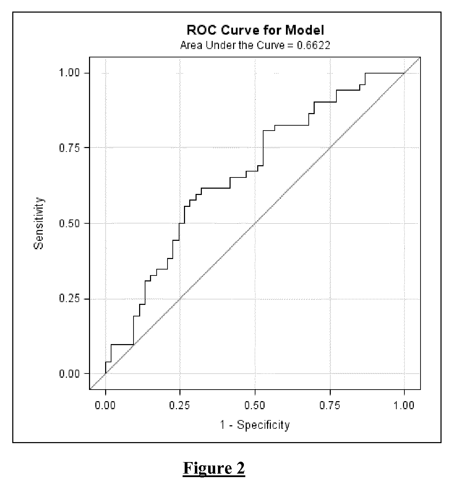

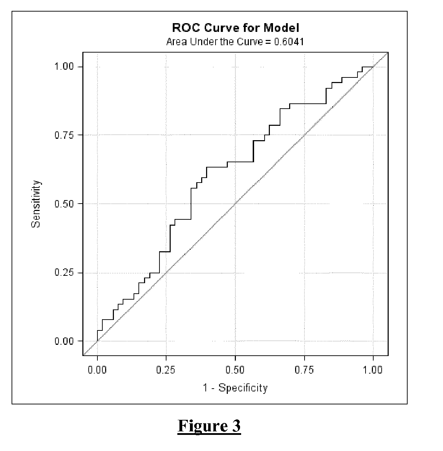

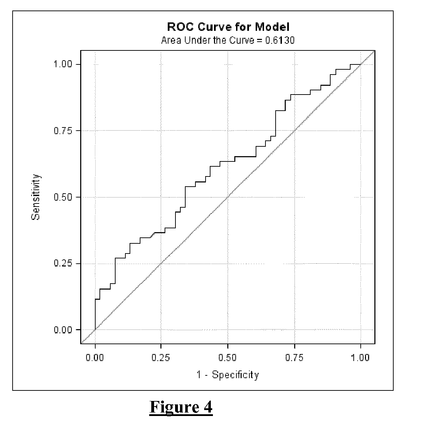

The inventors have measured the concentration of Aβ₁₋₄₀, Aβ₁₋₄₂, Aβₓ₋₄₀, Aβₓ₋₄₂, and Aβₓ₋₄₂/Aβₓ₋₄₀ ratios in plasma samples of genetically confirmed DM1 patients in comparison with controls. As illustrated in **examples** and **figures 1 to 4****,** this further led to the identification of Aβ levels thresholds, as biomarkers of Myotonic Dystrophy type 1 (DM1) disease, and more particularly the occurrence of brain dysfunction in an individual with Myotonic Dystrophy type 1 (DM1) disease.

According to the invention, a "brain dysfunction" refers to any neurodegenerative, neurodevelopmental and/or neurofunctional dysfunction targeting the brain and which may occur in an individual with DM1 disease. In particular, brain dysfunction includes frontal lobe dysfunction, visuoconstructive disability, anhedonia, difficulties to recognize facial emotion, daytime sleepiness, hypersomnolence, anxiety, apathy, avoidant personality trait, and difficulty for executive functions such as categorization, visual attention, cognitive flexibility or response inhibition. This would also include white matter lesions visualized by MRI (Itoh *et al.,* 2010) or neurofibrillary degeneration that could be observed with new PET (positron emission tomography) ligands (Maruyama *et al.,* 2013).

According to the invention, an *"individual with Myotonic Dystrophy type 1 (DM1) disease"* is an individual or group of individuals with a genomic DNA comprising at least one expanded DMPK allele comprising more than 50 CTG repeats, in particular more than 70 CTG repeats, which includes from 70 to more than 1000 CTG repeats, which includes 70 to 1300 CTG repeats.

DM1 may refer both to adult DM1 or to juvenile DM1.

According to the invention, a *"control patient"* or *"non-DM1"* individual is an individual or group of individuals with a genomic DNA comprising less than 50 CTG repeats, which includes between 5 to 37 CTG repeats in both *DMPK* alleles.

Of course, the man skilled in the Art will understand that the above-mentioned reference ranges must be determined using genetic testing according to reference ranges for allele sizes established by the Second International Myotonic Dystrophy Consortium (IDMC) or technical standards and guidelines for testing (International Myotonic Dystrophy Consortium 2000). For technical standards and guidelines for testing, the man skilled in the Art may also refer to Kamsteeg *et al.,* 2012 or Gonzalez *et al.,* 2000.

According to the invention, a *"blood sample"* refers either to whole blood, or to blood obtained after a fractionation step, as long as said fractionation step does not interfere with the detection of Aβ peptides. Blood fractionation steps are well known in the Art. Preferably, a blood sample of the invention is a blood plasma sample or a blood serum sample, and more preferably a blood plasma sample, which may be obtained after a blood plasma fractionation step. Blood plasma and blood serum are well known in the Art. For the purpose of the invention, the terms *"blood plasma"* and *"plasma",* or *"blood serum"* and *"serum"* will be considered as analogous. Thus, and as used herein, the term *"blood sample"* encompasses blood plasma as well as blood serum.

According to the invention, an *"Aβ peptide",* or *"amyloid-beta peptide",* also referred herein as *Aβ* or *Abeta,* refers to a set of polypeptides which are processed from the amyloid precursor protein (APP) of sequence SEQ ID NO 1 through sequential proteolytic cleavage by ß-secretase and γ-secretase enzymes, and are generally found as cleavage products of up to 43 amino acids in length, such as 40, 42 or 43 amino acids long.

As known in the Art, beta-amyloid protein 42 (also referred herein as Aβ₁₋₄₂) is derived from the amino acid sequence 672 to 713 of the longest human amyloid precursor protein comprising 770 amino acids (ref. P05067 of UniProt KB/ SwissProt A4-Human) of SEQ ID NO 1.

The total amount of Aβ peptides is also referred here below as *"Total"* or "Aβ_{Total}", which includes in particular Aβ₁₋₄₀, Aβ₁₋₄₂, Aβₓ₋₄₀ and Aβₓ₋₄₂, wherein X refers to the N-terminal end of the said Aβ peptide and corresponds to any amino acid between the N-terminal end and the C-terminal end of the Aβ full-length sequence, as long as it remains within the total number of amino acids of the said Aβ peptide, such as 40 for Aβ₁₋₄₀ and 42 for Aβ₁₋₄₂.

The total amount of Aβ peptides may further include Aβ_{1-Y} or Aβ_{X-Y}, wherein Y refers to the C-terminal end of the said Aβ peptide and corresponds to any amino acid between the N-terminal end and the C-terminal end of the Aβ full-length sequence. In particular, Y may range from 36 to 43.

More particularly, X may range from 1 to 17 and Y may range from 36 to 43 and in particular 36 to 42.

According to a more particular embodiment, Aβ_{Total} may be approximated by the sum of the contents in Aβ₁₋₄₀, Aβ₁₋₄₂, Aβₓ₋₄₀ and Aβₓ₋₄₂, wherein X is as defined above.

For instance, Aβ peptides can start at any of the amino acid comprised between 1 to 17 including the 3 and 11 pyroglutamylated variants.

The Aβ peptides from any of the starting amino acid can end at 36 until the 43 carboxy-terminal amino acid.

Aβ peptides are known to form long, insoluble and ordered fibers, also referred herein as *"amyloid deposits".* The core-component of such fibers is generally composed of Aβ peptides that are 42 residues in length.

There are several Aβ species differing from the amino- or carboxy-terminal part of Aβ. The carboxy-terminal species originate from the γ-secretase activity which progressively shorten the carboxy-terminal tail of Aβ. The most represented species is the Aβ₁₋₄₀ whereas Aβ₁₋₄₂ and shorter species have physiological plasma concentrations which may range below 40 pg/mL (Bilb *et al.,* 2012).

Aβ peptides are known in the Art and may be further processed at their N-terminal and/or C-terminal part. Of course, when Aβ peptides are further processed at their N- and/or C-terminal they may be shorter than 43 amino-acids long, depending on the position of the additional cleavage; such Aβ peptides are also considered by the invention.

An Aβ₁₋₄₀ peptide is a peptide consisting in the sequence 672 - 711 of SEQ ID NO 1. Thus an Aβ₁₋₄₀ peptide is a peptide of sequence SEQ ID NO 2.

An Aβ₁₋₄₂ peptide is a peptide consisting in the sequence 672 - 713 of SEQ ID NO 1. Thus an Aβ₁₋₄₂ peptide is a peptide of sequence SEQ ID NO 3.

Aβ peptides of the invention include Aβ₁₋₄₀ or Aβ₁₋₄₂ peptides which have been further processed at their N-terminal and/or C-terminal part. In particular, when Aβ₁₋₄₀ or Aβ₁₋₄₂ are processed at their N-terminal part they are also referred respectively as Aβₓ₋₄₀ and Aβₓ₋₄₂, wherein X refers to the N-terminal end of the peptide.

Alternatively, when Aβ₁₋₄₀ or Aβ₁₋₄₂ are processed at their C-terminal part they are also referred respectively as Aβ_{1-Y}, wherein Y refers to the C-terminal end of the peptide. In particular, the said Aβ peptide may thus stop at the Y carboxy-terminus position 36 to 43 (Takami *et al.,* 2009), which includes 36, 37, 38, 39, 40, 41, 42, and 43.

According to a particular embodiment, an *"Aβ peptide"* is selected in a group consisting of Aβ_{Total}, Aβ₁₋₄₀, Aβ₁₋₄₂, Aβₓ₋₄₀ and Aβₓ₋₄₂, wherein X can represent any integer, as long as it remains inferior to the total number of peptides comprised within the said Aβ peptide, such as 40 for Aβ₁₋₄₀ and 42 for Aβ₁₋₄₂.

Of note, Aβ peptides processed from Aβ₁₋₄₀ and Aβ₁₋₄₂ peptides and as short as 17 amino acids have been reported in the literature.

According to a more particular embodiment, an *"Aβ peptide"* is selected in a group consisting of Aβ_{Total}, Aβ₁₋₄₀, Aβ₁₋₄₂, Aβₓ₋₄₀ and Aβₓ₋₄₂, wherein X ranges from 1 to 17, which includes 1,2, 3, 4, 5, 6, 7, 8, 9, 10, 11, 12, 13, 14, 15, 16 and 17. For instance, X may be equal to 1, 10, 11, 12, 13 or 17.

According to a preferred embodiment, an *"Aβ peptide"* is selected in a group consisting of Aβ₁₋₄₀, Aβ₁₋₄₂, Aβₓ₋₄₀ and Aβₓ₋₄₂, wherein X ranges from 1 to 17, which includes 1, 2, 3, 4, 5, 6, 7, 8, 9, 10, 11, 12, 13, 14, 15, 16 and 17. For instance, X may be equal to 1, 10, 11, 12, 13 or 17.

Alternatively, an *"Aβ peptide"* is selected in a group consisting of Aβ₁₋₄₀ and Aβ₁₋₄₂.

As used herein, an *"amount"* of Aβ peptide refers to a quantification value of an Aβ peptide in a sample and thus encompasses the amount or concentration of an Aβ peptide that is contained in the said sample as well as the amount or concentration of an Aβ peptide in the said sample. The amount value of an Aβ peptide may be expressed in a variety of quantification units, which encompasses arbitrary units and conventional units such as weight units, molar units or concentration units.

The invention relates to an *in vitro* method for detecting the occurrence of Myotonic Dystrophy type 1 (DM1) disease in an individual, comprising the steps of:
a) providing a blood sample from said individual,
b) measuring an amount of Aβ peptide within said blood sample,
c) comparing the amount measured at step b) with a reference value,
d) determining the occurrence of DM1 in said individual from the comparison performed at step c).

The *in vitro* method above may also be defined as comprising a step a) comprising measuring an amount of Aβ peptide within a blood sample previously collected from an individual, and comprising steps b) and c) consisting of steps c) and d) of the above method, respectively.

Thus, the invention also relates to an *in vitro* method for detecting the occurrence of Myotonic Dystrophy type 1 (DM1) disease in an individual, comprising the steps of:
a) measuring an amount of Aβ peptide within a blood sample previously collected from said individual,
b) comparing the amount measured at step a) with a reference value,
c) determining the occurrence of DM1 in said individual from the comparison performed at step b).

In determining the occurrence of Myotonic Dystrophy type 1 (DM1) disease in an individual, the invention further relates to the detection of the occurrence of brain dysfunction in an individual with Myotonic Dystrophy type 1 (DM1) disease.

Thus a first aspect of the invention is to provide an *in vitro* method for detecting the occurrence of brain dysfunction in an individual with Myotonic Dystrophy type 1 (DM1) disease, comprising the steps of:
a) providing a blood sample from said individual,
b) measuring an amount of Aβ peptide within said blood sample,
c) comparing the amount measured at step b) with a reference value,
d) determining the occurrence of brain dysfunction in said individual from the comparison performed at step c).

The *in vitro* method above may also be defined as comprising a step a) comprising measuring an amount of Aβ peptide within a blood sample previously collected from an individual, and comprising steps b) and c) consisting of steps c) and d) of the above method, respectively.

Thus, the invention also relates to an *in vitro* method for detecting the occurrence of brain dysfunction in an individual with Myotonic Dystrophy type 1 (DM1) disease, comprising the steps of:
a) measuring an amount of Aβ peptide within a blood sample previously collected from said individual,
b) comparing the amount measured at step a) with a reference value,
c) determining the occurrence of brain dysfunction in said individual from the comparison performed at step b).

When the amount of Aβ peptide from the blood sample of said individual is increased compared to a reference value, it is indicative of the occurrence of DM1 disease, and more particularly of the occurrence of brain dysfunction in an individual having DM1.

Thus the invention relates to the amount of Aβ peptide within a blood sample which is used as a biomarker for the occurrence of DM1 disease in an individual, and more particularly the occurrence of brain dysfunction in an individual with Myotonic Dystrophy type 1 (DM1) disease.

Thus the invention also relates to methods, such as the ones described above and here below, wherein the blood sample is selected in a group comprising a plasma sample and a serum sample.

It is recommended that the blood sample is collected in a tube that is not a glass tube (see Material & Methods for reference). Preferably, the blood sample is collected in a polyethylene tube, and even more preferably a Na⁺-EDTA polyethylene tube.

According to an exemplary embodiment, the invention relates to an *in vitro* method for detecting the occurrence of brain dysfunction in an individual with Myotonic Dystrophy type 1 (DM1) disease, comprising the steps of:
a) providing a plasma sample from said individual,
b) measuring an amount of Aβ peptide within said plasma sample,
c) comparing the amount measured at step b) with a reference value,
d) determining the occurrence of brain dysfunction in said individual from the comparison performed at step c).

The *in vitro* method above may also be defined as comprising a step a) comprising measuring an amount of Aβ peptide within a blood sample previously collected from an individual, and comprising steps b) and c) consisting of steps c) and d) of the above method, respectively.

Thus, the invention also relates to an *in vitro* method for detecting the occurrence of brain dysfunction in an individual with Myotonic Dystrophy type 1 (DM1) disease, comprising the steps of:
a) measuring an amount of Aβ peptide within a plasma sample previously collected from said individual,
b) comparing the amount measured at step a) with a reference value,
c) determining the occurrence of brain dysfunction in said individual from the comparison performed at step b).

According to a second aspect, the invention relates to an *in vitro* method for discriminating between DM1 and a non-DM1 neurodegenerative disease in an individual, comprising the steps of:
a) providing a blood sample from said individual,
b) measuring an amount of Aβ peptide within said blood sample,
c) comparing the amount of Aβ peptide within said blood sample with a reference value,
d) discriminating between Myotonic Dystrophy type 1 (DM1) disease and a non-DM1 neurodegenerative disease from the comparison performed at step c).

The *in vitro* method above may also be defined as comprising a step a) comprising measuring an amount of Aβ peptide within a blood sample previously collected from an individual, and comprising steps b) and c) consisting of steps c) and d) of the above method, respectively.

Thus, the invention also relates to an *in vitro* method for discriminating between DM1 and a non-DM1 neurodegenerative disease in an individual, comprising the steps of:
a) measuring an amount of Aβ peptide within a blood sample previously collected from said individual,
b) comparing the amount measured at step a) with a reference value,
c) discriminating between Myotonic Dystrophy type 1 (DM1) disease and a non-DM1 neurodegenerative disease from the comparison performed at step b).

In particular, a non-DMI neurodegenerative disease is selected in a group comprising: Alzheimer's disease, frontolobar degeneration, Lewy body dementia, Parkinson's disease, Tauopathies or Synucleopathies characterized respectively by the aggregation of tau or synuclein. None of those neurological diseases are associated with instable expansion of CTG repeats in the *DMPK* gene.

According to the invention, *"measuring an amount of Aβ peptide"* can be achieved using any method known in the Art, such as the methods described in Lachno *et al.* (Lachno, D. R., Evert, B. A., Vanderstichele, H., Robertson, M., Demattos, R. B., Konrad, R. J., et al. (2013). Validation of assays for measurement of amyloid-β peptides in cerebrospinal fluid and plasma specimens from patients with Alzheimer's disease treated with solanezumab. Journal of Alzheimer's disease: JAD, 34(4), 897-910).

In particular, the amount of Aβ peptide in a blood sample can be measured by bringing into contact the sample with an Aβ peptide binding molecule. An Aβ peptide binding molecule may refer to any molecule which is able to bind to either one of the Aβ peptides of the invention, and for which the presence may be further detected either alone or as a complex with said Aβ peptide. For instance, an Aβ peptide binding molecule may be an aptamer or an antibody, particularly an antibody, directly or indirectly labeled with a detectable molecule. When the Aβ peptide binding molecule is an antibody, it may be a monoclonal or a polyclonal antibody. Aβ peptide binding molecules and detectable molecules are well known in the Art.

More particularly, an Aβ peptide binding molecule may be an antibody selected from the INNO-BIA plasma Aβ form test (Innogenetics®), and/or an antibody that is directed against an Aβ peptide of the invention, which includes an Aβ peptide chosen in a list consisting of: Aβ₁₋₄₀, Aβ₁₋₄₂, Aβₓ₋₄₀, Aβₓ₋₄₂, Aβ_{1-Y} and Aβ_{X-Y}, wherein X and Y are as defined above.

More particularly, an Aβ peptide binding molecule may be an antibody that is directed against an Aβ peptide chosen in a list consisting of Aβ₁₋₄₀, Aβ₁₋₄₂, Aβₓ₋₄₀ and Aβₓ₋₄₂, wherein X is preferably ranging from 1 to 17.

Even more particularly, an Aβ peptide binding molecule may be an antibody directed against a polypeptide of sequence SEQ ID NO 2 or SEQ ID NO 3.

Other examples of such antibodies include the monoclonal antibody 4G8 (Covance, Ref. SIG-39245) which is directed against a polypeptide comprised between amino acids 17 and 24 of a sequence of SEQ ID NO 2 or SEQ ID NO 3, and more particularly comprised between amino acids 18 and 22.

Alternatively an antibody may be directed against a polypeptide comprised between amino acids 1 and 18, such as antibodies from the INNO-BIA kit.

According to a particular embodiment, the amount of Aβ peptide in a blood sample is measured by an ELISA method, or *Enzyme-Linked ImmunoSorbent Assay.*

According to an exemplary embodiment, the amount of Aβ peptide in a blood sample is measured using ELISA variants, such as the multiplex Luminex xMAP^{®} technology as described in the Material & Methods, or the Meso Scale Discovery (MSD®) assay (Meso-Scale).

According to a preferred embodiment, the invention relates to methods which include a step of measuring an amount of Aβ peptide in plasma or serum, and preferably plasma. Thus, the blood sample is preferably a plasma sample.

When the invention relates to methods for detecting the occurrence of Myotonic Dystrophy type 1 (DM1) disease in an individual, for detecting the occurrence of brain dysfunction in an individual with DM1 or alternatively for discriminating between DM1 and a non-DM1 neurodegenerative disease in an individual, a *"reference value"* or *"first reference value"* refers to the amount of said Aβ peptide within a blood sample from a control patient not having a DM1 disease. Reference values which have been considered as physiological for one set of Aβ peptides are described in the Art (see for instance Bilb *et al.,* 2012).

According to an exemplary embodiment reference values of Aβ peptides within a blood sample for non-DM1 patients are further disclosed in the examples and **Table 2.** Preferably, reference values for Aβ peptides are as defined here below:
**Aβ₁₋₄₀=** 120.69 pg/mL,
**Aβ₁₋₄₂=** 39.75 pg/mL,
**Aβₓ₋₄₀=** 172.25 pg/mL,
**Aβₓ₋₄₂=** 33.00 pg/mL,
wherein X is an integer equal or inferior to 40 for Aβₓ₋₄₀ and 42 for Aβₓ₋₄₂, and preferably ranges from 1 to 17.

In view of the above, when the amount of Aβ peptide is higher than the reference value, it is indicative of the occurrence of Myotonic Dystrophy type 1 (DM1) disease in an individual, and more particularly of the occurrence of brain dysfunction in an individual with Myotonic Dystrophy type 1 (DM1) disease.

It will be clear for the man skilled in the Art that the above-mentioned reference values may be compared in light of a number of factors, such as pre-analytical, analytical or assay-related factors, in order to minimize variability due to bias and/or imprecision and to improve the consistency among laboratories.

In particular, variability may be caused both by differences between laboratories and differences between manufactured lots.

More particularly, the reference values should be adapted according to the centers where the dosages are made, to the tubes and plastic ware used and further corrected by comparing the values of a group of controls and DM1 patients in order to reach statistical significance. For instance, said dosages may be corrected by comparing the value of a group of 30 controls and 30 DM1 patients.

In view of the above, the variability of measurements between laboratories remains too high to provide definite cut-off values, and it is therefore recommended that each laboratory determines internally its own reference values (see also Mattson *et al.,* 2013 for the determination of between-laboratory **vs.** between-lot variability in the context of Aβ₁₋₄₂ measurement in the cerebrospinal fluid for Alzheimer's disease research).

The measurement of the amount of Aβ peptides from a blood sample may thus provide useful information on an individual having a diagnosed DM1, or suspected of having DM1, in which case methods of the invention will be advantageously combined with the assessment of other biomarkers.

In view of the above, methods of the invention may further comprise a step of measuring the ratio between at least two different Aβ peptides selected in a group comprising, and preferably consisting in: Aβ₁₋₄₀, Aβ₁₋₄₂, Aβₓ₋₄₀ and Aβₓ₋₄₂, wherein X is as defined above.

Alternatively, the method may comprise a step of measuring the ratio between at least one Aβ peptide as defined above and the total amount of Aβ peptides, also referred here below as *"Total"* or "Aβ_{Total}", which includes Aβ₁₋₄₀, Aβ₁₋₄₂, Aβₓ₋₄₀ and Aβₓ₋₄₂, wherein X is as defined above.

Measuring the *total* amount of Aβ peptides can be achieved using at least one antibody, preferably more than one antibody, which recognizes specifically an epitope common to all Aβ peptides of the invention, such as an epitope comprised within the regions 17-40 and 17-42 of N-terminally processed Aβ₁₋₄₀ and 17-42 of N-terminally processed Aβ₁₋₄₂ peptides. For example, a first antibody may recognize a first epitope consisting in sequence 17-25 of a polypeptide of sequence SEQ ID NO 3; a second antibody may recognize a second epitope consisting in sequence 35-42 of a polypeptide of sequence SEQ ID NO 3.

Thus, methods of the invention may comprise a step of measuring the ratio between at least two different Aβ peptides, wherein said ratio is selected in the group comprising, and preferably consisting in Aβ₁₋₄₂/Aβ₁₋₄₀, Aβₓ₋₄₂/Aβₓ₋₄₀, Aβ₁₋₄₂/Aβ_{Total}, Aβ₁-₄₂/Aβ_{Total}, Aβₓ₋₄₀/Aβ_{Total} and Aβₓ₋₄₂/AβTotal.

According to the invention, the ratio between Aβ peptides of the invention should not vary when compared to a reference value from control patients, in contrast to non-DM1 neurodegenerative diseases selected in a list comprising: Alzheimer disease, Depression, acute cerebral ischemia, conversion to Alzheimer's disease for Down syndrome (see Piccinni *et al.,* 2013; Schupf *et al.,* 2010; Bibl *et al.,* 2012).

Advantageously, methods of the invention may also comprise a step of determining the CTG repeat number in said individual's genomic DNA. Genomic DNA may be extracted from whole blood according to standard protocols.

According to the invention, an individual is diagnosed as a DM1 patient when the individual's genomic DNA comprises at least one expanded *DMPK* allele of at least 50 CTG repeats, in particular over 70 CTG repeats, which includes 70 to 1300 CTG repeats. Thus, methods of the invention may also comprise a step of measuring the number of CTG repeats on at least one *DMPK* allele from said individual.

Assessment of the number of CTG repeats is known in the Art and is further described in the Material & Methods section.

According to a particular embodiment, the CTG repeat number of the expanded alleles may be determined with a long-PCR assay (as described in Sergeant *et al.,* 2001 or in Dhaenens *et al.,* 2011; or as described in the Material & Methods section) and/or Southern blot analysis according to the international myotonic dystrophy consortium guidelines (IDMC, 2010).

Methods of the invention may also comprise a step of measuring an amount of Aβ peptide within a cerebrospinal fluid (CSF) sample from an individual having, or suspected of having a DM1 disease.

According to said embodiment, a method of the invention may comprise a step of comparing the amount of an Aβ peptide within said CSF sample to a second reference value.

Assessment of an amount of Aβ peptide within a CSF sample and within a blood sample may be achieved either with the same Aβ peptide or with a different Aβ peptide, and preferably with the same Aβ peptide.

According to said embodiment, said *"second reference value"* refers to the amount of Aβ peptide within a CSF sample from one individual or group of individuals not having a DM1 disease. Reference values for the amount of Aβ peptide within CSF samples can be found in the Art (see Winblad *et al.,* 2008; Peric *et al.,* 2013).

When the amount of a first Aβ peptide within a blood sample is higher than the first reference value, and the amount of a second Aβ peptide within a CSF sample is lower than a second reference value, it is indicative of the occurrence of DM1 in an individual, and more particularly the occurrence of brain dysfunction in an individual having a DM1 disease.

More particularly, the absence of variation of the ratio between Aβ peptides in an individual, such as in blood samples and CSF samples, and more particularly blood samples, is indicative of the occurrence of DM1 in said individual, and more particularly the occurrence of brain dysfunction in an individual having said DM1 disease.

Of note, reduced levels of Aβ₁₋₄₂ in the CSF are associated with several neurodegenerative disorders (see Peric *et al.,* 2013). In particular, it has been noted that the magnitude of Aβ₁₋₄₂ difference in the CSF of DM1 patients in relation to normal control patients is small in comparison with changes noted in Alzheimer's Disease (AD) and in Amyotrophic Lateral Sclerosis (ALS), but comparable with changes seen in Creutzfeldt-Jakob disease (CJD), Multiple System Atrophy (MSA), Fronto-Temporal Dementia (FTD) and Progressive Supranuclear Palsy (PSP).

Thus, according to an alternative embodiment, the invention also relates to an *in vitro* method for discriminating between DM1 and a non-DM1 neurodegenerative disease in an individual, comprising the steps of:
a) providing a blood sample and a CSF sample from said individual,
b) measuring an amount of Aβ peptide within each of said blood sample and said CSF sample,
c) comparing the amount of (i) Aβ peptide within said blood sample with a first reference value, and the amount of (ii) Aβ peptide within said CSF sample with a second reference value,
d) discriminating between Myotonic Dystrophy type 1 (DM1) disease and a non-DM1 neurodegenerative disease from the comparison performed at step c).

The *in vitro* method above may also be defined as comprising a step a) comprising measuring an amount of Aβ peptide within a blood sample previously collected from an individual and within a CSF sample previously collected from the said individual, and comprising steps b) and c) consisting of steps c) and d) of the above method, respectively.

Thus, the invention also relates to an *in vitro* method for discriminating between DM1 and a non-DM1 neurodegenerative disease in an individual, comprising the steps of:
a) measuring an amount of Aβ peptide within each of said blood sample and said CSF sample,
b) comparing the amount of (i) Aβ peptide within said blood sample with a first reference value, and the amount of (ii) Aβ peptide within said CSF sample with a second reference value,
c) discriminating between Myotonic Dystrophy type 1 (DM1) disease and a non-DM1 neurodegenerative disease from the comparison performed at step b).

When the amount of a first Aβ peptide within a blood sample is lower than the first reference value, and/or the amount of a second Aβ peptide within a CSF sample is higher than a second reference value, it is indicative of the occurrence of a non-DM1 neurodegenerative disease.

As already discussed in the present specification, and in the light of the inventors results described herein, it cannot be excluded that the reduced concentration of CSF Aβ peptides in DM1 would result from an increased clearance through the blood brain barrier thus potentially modifying plasma Aβ concentrations in DM1 patients.

Thus, any reversal of this leakage would be indicative of a successful treatment toward the recovery of the BBB impermeability and therefore normalization of plasma Aβ concentration may be informative for theragnostic purposes.

Our present results altogether suggest that Aβ species plasma concentration could also serve as a predictive biomarker for determining the efficacy of a treatment of Myotonic Dystrophy type 1 (DM1) disease in an individual.

Thus, according to a third aspect, the invention relates to an *in vitro* method for determining the efficacy of a treatment of Myotonic Dystrophy type 1 (DM1) disease in an individual, comprising the steps of:
a) providing a blood sample from said individual,
b) measuring an amount of Aβ peptide within said blood sample,
c) comparing the amount measured at step b) with a reference value,
d) determining the efficacy of said treatment in said individual from the comparison performed at step c).

The *in vitro* method above may also be defined as comprising a step a) comprising measuring an amount of Aβ peptide within a blood sample previously collected from an individual, and comprising steps b) and c) consisting of steps c) and d) of the above method, respectively.

Thus, the invention also relates to an *in vitro* method for determining the efficacy of a treatment of Myotonic Dystrophy type 1 (DM1) disease in an individual, comprising the steps of:
a) measuring an amount of Aβ peptide within said blood sample,
b) comparing the amount measured at step a) with a reference value,
c) determining the efficacy of said treatment in said individual from the comparison performed at step b).

According to the said embodiment, when the method is for determining the efficacy of a treatment of Myotonic Dystrophy type 1 (DM1) disease in an individual, the *"reference value"* or *"first reference value"* refers to the amount of Aβ peptide in a blood sample from said individual which has been collected prior to the administration of said treatment. Thus, an amount of Aβ peptide which is lower than said reference value is indicative of the efficacy of said treatment, whereas an amount which is higher or equal is indicative of a lack of efficacy of said treatment.

According to a fourth aspect, the invention relates to an assay for the screening of a candidate agent useful in the treatment of DM1 in an individual, said assay comprising determining whether a tested agent can modulate, such as increase or reduce and preferably reduce, the level of an Aβ peptide in a blood sample from said individual.

In particular, the invention relates to an assay for the screening of a candidate agent useful in the treatment of brain dysfunction in an individual with DM1, as defined above.

According to the invention, a candidate agent is predicted to be useful in the treatment of DM1, and more particularly the treatment of brain dysfunction in an individual with DM1 if, after its administration, the level of Aβ peptide in said blood is reduced. A candidate agent is predicted as not useful in the treatment of DM1, and more particularly the treatment of brain dysfunction in an individual with DM1 if, after its administration, the level of Aβ peptide in said blood is unchanged, or alternatively if the level of Aβ peptide in said blood is increased.

### EXAMPLES

### A. MATERIAL & METHODS

### 1. Participants

A total of 103 patients (52 controls and 61 DM1 patients) were included among those regularly followed at the "Centre de Référence des maladies Neuromusculaires", the Department of Neurology of the Centre Hospitalier Regional et Universitaire of Lille. The group of control individuals corresponded to patients received at the Neurology Department for a suspicion of myotonic dystrophy but myotonic dystrophy was ruled out after molecular genetic analysis. The control patients showed no other neurological symptoms or any disease that would justify excluding them from the study. For DM1 patients, the diagnosis was first established by clinical assessments and further confirmed by molecular analysis of the *DMPK* gene. The clinical protocol was submitted for ethical approval to the Federation of Clinical Research of Regional University Hospital Centre. All patients and controls were aged of 17 years old and over and genetic diagnosis and Aβ plasma dosage were performed using the same blood sample and inform consents were obtained.

### 2. Molecular genetic diagnosis

Non-fasting blood was collected by venipuncture in Na⁺-EDTA polyethylene tubes according to the recommendations (Perret-Liaudet *et al.,* 2012) and plasma was separated from blood cells after centrifugation. Genomic DNA was extracted from blood cells by standard procedures. The CTG repeat number in the *DMPK* gene was determined for the whole 103 subjects by polymerase chain reaction (PCR). The CTG repeat number of the expanded alleles was determined with a long-PCR assay (Sergeant *et al.,* 2001) and/or Southern blot analysis according to the international myotonic dystrophy consortium guidelines (IDMC, 2010).

### 3. Plasma amyloid dosage

Plasma amyloid concentrations were determined using the multiplex Luminex xMAP^{®} technology. The plasma was recovered from the blood samples used to perform the genetic diagnosis of DM1. Following centrifugation to recover blood cells for genetic analyses, the plasma was transfered in polypropylene tubes and stored at -80°C pending the Aβ dosage. The plasma sample was frozen once and thawed only once for biochemical analyses. The plasma Aβ peptide assay was performed using the INNO-BIA plasma Aβ modules A and B (Innogenetics, Ghent, Belgium) for the dosage of Aβ₁₋₄₀, Aβ₁₋₄₂ or amino-terminal truncated Aβ species Aβₓ₋₄₀, Aβₓ₋₄₂, respectively. The dosage was performed using 25 µl of plasma according the manufacturer's instructions. Measurement of Aβ concentration was performed on a multiplex xMAP technique with a LABScan-100 system (Luminex BV, The Netherlands). Concentrations were reported on an Excel table for further statistical analyses. Concentrations of Aβ peptides are given as pg/mL.

### 4. Long-PCR

Genomic DNA was prepared from brain tissue by phenol-chloroform extraction protocol. The genetic diagnosis of DM1 was confirmed by PCR amplification of the 3'UTR *DMPK* region (Cheng et al., 1996) using an Expand Long Template PCR System™ kit (Roche Diagnostics GmbH, Mannheim, Germany).

Each 50 µl reaction consisted of 1 × Expand Long PCR Buffer; 0.35 mM each of dATP, dCTP, and dTTP; 0.23 mM dGTP; 0.12 mM 7-deaza dGTP; 0.4 µM of primers DMPK1 of SEQ ID NO 4 (5'-CACAGGCTGAAGTGGCAGTTCCA-3') and DMPK2 of SEQ ID NO 5 (5'-TGTCGGGGTCTCAGTGCATCCA-3'); 3.5 U of Taq DNA polymerase; 1.75 mM MgCl₂; and 0.5, 2 and 10 ng of total genomic DNA. Amplifications were performed in a PTC200 thermocycler (MJ Research, Prolabo, France). Following denaturation for 5 min at 95°C, 35 cycles of amplification were performed: denaturation at 95°C for 1 min, annealing at 62°C for 30 s, and extension at 68°C for 10 min. PCR products were resolved on 1% agarose gels.

Southern blot transfer and probe-mediated detection were carried out as follows. Agarose gels were denatured in 0.4 M NaOH and 0.6 M NaCl and rinsed twice in 2× SSC before being transferred to Hybond N+ nylon membranes (G&E Healthcare) Blots were pre-hybridized in DIG Easy Hyb solution (Roche) for 30 min at 59 °C. Hybridization was performed in a fresh solution containing 100 pmol 3'-DIG-(CTG)10 (DIG Oligonucleotide Terminal 3' Labeling Kit; Roche) for 2 h at 59 °C, followed by 2 washes in 2× SSC, 0.1% SDS at room temperature for 5 min, and 2 washes in 0.5× SSC, 0.1% SDS for 15 min at 59 °C. Probe binding was detected using a DIG Luminescence Detection Kit (Roche) according to the manufacturer's instructions (Amersham-Pharmacia Biotech, Orsay, France). Film exposure times ranged from 5 to 15 min. DNA molecular weight standards were loaded onto each agarose gel (DNA Marker IV; Boehringer Mannheim GmbH, Germany).

### 5. Statistical methods

Quantitative data are presented as mean with standard deviation. Qualitative data were compared with the χ2 tests. Quantitative data were compared using the SAS software release 9.01 (SAS Institute INC, Cary, NC). Cases with DM and controls were compared in terms of plasma Aβ concentrations or their ratio in an analysis of covariance (ANCOVA) that adopted a general linear model (GLM procedure) adjusted (or not) for age, and gender. A logistic regression model was used to estimate the association between plasma Aβ concentrations or their ratio and the risk of DM by entering the variable as linear terms. Furthermore, tertiles were defined and the lowest was used as a reference. Significance levels were set at p<0.05. Analyses were performed with the SAS software release 9.02 (SAS Institute INC, Cary, NC)

### B. RESULTS

### 1. Variation of Aβ peptides in plasma in DM1 patients vs controls

The control population included 52 participants genetically confirmed to have two normal *DMPK* alleles (CTG repeat number below 25). This group was composed of 26 women and 26 men aged of 17 to 83 years (mean ± SD, 41±3 yrs) and the mean age was not significantly different between women and men (40 ± 17 yrs vs 43 ± 16 yrs). The genetic diagnosis of DM1 was confirmed in 61 patients (43 women and 18 men) aged between 17 and 67 years old (38 ± 2 yrs), not significantly different between genders and not different also from the control group **(Table 1).** In the DM1 group, the average size of the expansion was of 563 ± 341 CTG repeats for men and 569 ± 307 CTG repeats for women. The average of repeat sizes was not significantly different between genders **(Table 1).** Noteworthy, all DM1 patients had one expanded allele with a minimum of 70 to a maximum 1300 CTG repeats. The second *DMPK* allele was normal.

**Table 1: Controls and DM1 patients included in the study**

| | ***Gender*** | ***Number*** | ***Age, mean (SD)*** | ***Allele size, mean (SD)*** |
|---|---|---|---|---|
| ***Controls*** | | | | |
| | Women | n=27 (50.9%) | 39.9 (16.9) | 13 (5) |
| | Men | n=26 (40.1%) | 42.7 (15.6) | 12 (4) |
| | | | | |
| | *Total* | *n=53* | *41.3 (16.1)* | *12 (4)* |

| ***DM1 patients*** | | | | |
|---|---|---|---|---|
| | Women | n=34 (65.4%) | 38.4 (13.8) | 569 (307) |
| | Men | n=18 (34.6%) | 37.6 (11.7) | 563 (341) |
| | | | | |
| | *Total* | *n=52* | *38.2 (13.0)* | *567 (316)* |

Plasma Aβ concentrations were determined using the multiplex InnoBia Assay kit enabling to measure several concentrations simultaneously and within the same plasma aliquot. The Aβ species measured comprised the Aβ₁₋₄₀, Aβ₁₋₄₂ and Aβₓ₋₄₀, Aβₓ₋₄₂ **(Table 2).** The plasma Aβ₁₋₄₀ and Aβ₁₋₄₂ concentrations were significantly higher in DM1 patients thanAβ₁₋₄₀ and Aβ₁₋₄₂ concentrations measured in the plasma of controls (Controls Aβ₁₋₄₀ 142.7 ± 51.4 ng/mL, p = 0.014; Aβ₁₋₄₂ 36.2 ± 10.7 ng/mL, p=0.004 versus DM1 patients Aβ₁₋₄₀ 166.8 ± 47.4 ng/mL, Aβ₁₋₄₂ 42.3 ± 10.3 ng/mL, **Table 2).**

**Table 2: Plasma Amyloid-beta peptide concentrations in pg/mL**

| | ***Controls*** | ***DM1 patients*** | ***Crude p*** | ***Gender and Age adjusted p*** |
|---|---|---|---|---|
| **Aβ**_{**1**-}**₄₀, m (SD)** | 142.7 (51.4) | 166.8 (47.4) | **0.014** | **0.015** |
| **Aβ₁₋₄₂, m (SD)** | 36.2 (10.7) | 42.3 (10.3) | **0.004** | **0.006** |
| **Aβ₁₋₄₀/ Total, m (SD)** | 0.371 (0.066) | 0.388 (0.050) | 0.14 | 0.18 |
| **Aβ₁₋₄₂/ Total, m (SD)** | 0.096 (0.019) | 0.100 (0.016) | 0.32 | 0.49 |
| **Aβ₁₋₄₂/ Aβ₁₋₄₀, m (SD)** | 0.266 (0.062) | 0.260 (0.049) | 0.61 | 0.48 |
| | | | | |
| **Aβₓ₋₄₀, m (SD)** | 169.1 (41.6) | 184.3 (44.8) | 0.07 | **0.04** |
| **Aβₓ₋₄₂, m (SD)** | 29.5 (6.6) | 33.0 (7.8) | **0.017** | **0.015** |
| **Aβₓ₋₄₀**/ **Total, m (SD)** | 0.452 (0.072) | 0.433(0.053) | 0.13 | 0.19 |
| **Aβₓ₋₄₂/ Total, m (SD)** | 0.080 (0.017) | 0.078 (0.015) | 0.56 | 0.57 |
| **Aβₓ₋₄₂**/ **Aβₓ₋₄₀, m (SD)** | 0.181 (0.046) | 0.183 (0.043) | 0.77 | 0.92 |

Of note, the increased Aβ₁₋₄₂ concentration was more significantly elevated than the raised concentration of Aβ₁₋₄₀ in the blood plasma of DM1 patients.

Several amino-truncated Aβ species are also produced from APP metabolism and the plasma concentrations of Aβₓ₋₄₀ and Aβₓ₋₄₂ therefore measured. The plasma concentrations Aβₓ₋₄₂ significantly rose above those of control cases (Controls Aβₓ₋₄₂ 29.5 ± 6.6 ng/mL versus DM1 patients Aβₓ₋₄₂ 33.0 ± 7.8 ng/mL, p=0.017 **Table 2)** whereas the Aβₓ₋₄₀ concentration was significantly higher in DM1 patients only following adjustment for gender and age (Controls Aβₓ₋₄₀ 169.1 ± 41.6 ng/mL versus DM1 184 ± 44.8 ng/mL, p=0.04). For Aβ₁₋₄₀, Aβ₁₋₄₂ and Aβₓ₋₄₂, differences of the concentrations between DM1 patients and controls all remained significant following adjustment for age and sex.

The ratios between Aβ species have been also compared since those may be indicative of a modification of the protease activity generating the carboxy-terminal extremity of Aβ peptides. The ratios between Aβ species were unchanged between controls and DM1 patients whatever the ratio considered, between individual species upon the total Aβ or Aβ₄₂ toward Aβ₄₀ concentrations **(Table 2).** Together, those results show that Aβ plasma concentration in DM1 rises more significantly for Aβ₄₂ species without significant modification of the ratio between Aβ species.

In order to determine if the increased plasma concentrations could be associated with the number of CTG, Pearson and the Spearman rank statistical test have been applied. Plasma concentrations of Aβ peptides were independent of "normal" CTG allele in the control cohort. Changes in plasma Aβ concentrations of DM1 patients were not correlated to the number of CTG repeats although an inverse correlation was observed for Aβ₁₋₄₀ (r Spearman of -0.23, p=0.07) within the DM1 group, suggesting that longer expanded CTG alleles may rather be associated with a reduced plasma Aβ₁₋₄₀ concentration **(Table 3).**

**Table 3: Correlation between the number of CTG repeats and plasma Aβ concentrations and ratio**

| | ***Controls*** | | | ***DM1*** |
|---|---|---|---|---|
| | ***r Pearson, p*** | ***r Spearman*** | ***r Pearson, p*** | ***r Spearman*** |
| **Aβ₁₋₄₀** | 0.007 (p=0.95) | -0.05 (p=0.70) | -0.21 (p=0.11) | **-0.23 (p=0.07)** |
| **Aβ₁₋₄₂** | 0.05 (p=0.70) | -0.05 (p=0.71) | -0.11 (p=0.41) | -0.15 (p=0.26) |
| **Aβ₁₋₄₂/Aβ₁₋₄₀** | 0.03 (p=0.79) | 0.05 (p=0.68) | 0.08 (p=0.54) | 0.04 (p=0.76) |
| | | | | |
| **Aβₓ₋₄₀** | -0.15 (p=0.23) | -0.09 (p=0.46) | 0.04 (p=0.74) | 0.08 (p=0.54) |
| **Aβₓ₋₄₂** | -0.11 (p=0.38) | -0.03 (p=0.81) | -0.02 (p=0.87) | -0.02 (p=0.86) |
| **Aβₓ₋₄₂/Aβₓ₋₄₀** | 0.02 (p=0.90) | 0.02 (p=0.85) | -0.07 (p=0.62) | 0.06 (p=0.68) |

A stratification of the CTG repeat length has been applied in order to determine whether there is or not a correlation between the length of the mutation and Aβ plasma concentrations. No significant correlation was found.

Thus we here report that there is an increased concentration of Aβ peptides in the plasma of DM1 patients that is significantly associated with the disease and not with other parameters

### 2. Determination of ROC curves for sensitivity/specificity

ROC curves **(see** **figures 1 to 4****)** have been determined according to the standard statistical methods which have been described in the Material & Methods section.

Logistic regression has been undergone and odds ratios (OR) have been determined as follows:

**Table 4: Logistic Regression and Odds Ratios (OR) adjusted for age and gender without Bonferroni correction**

| | ***OR***(Per SD*** | ***p**** | | | | |
|---|---|---|---|---|---|---|
| | ***[CI95]**** | | **SD** | **AUC** [CI95%] | **P/AUC = 0.50** | **P/AUC age** |
| **Aβ₁₋₄₀, m(SD)** | 1.68 [1.09-2.59] | **0.018** | **50.71** | **0.64 [0.54-0.74]** | **0.009** | **0.23** |
| | | | | | | |
| **Aβ₁₋₄₂, m(SD)** | 1.80 [1.17-2.78 | **0.008** | **10.85** | **0.66 [0.56-0.77]** | **0.0023** | **0.12** |
| **Aβ_{x-40,} m(SD)** | 1.55 [1.01-2.38] | 0.04 | 43.67 | 0.60 [0.50-0.71] | 0.06 | 0.53 |
| **Aβₓ₋₄₂, m(SD)** | 1.67 [1.10-2.55] | **0.017** | **7.39** | **0.61 [0.51-0.72]** | **0.04** | **0.44** |

| | | | | | | |
|---|---|---|---|---|---|---|
| * **adjusted for age and gender** | | | | | | |

Best outcomes between sensibility and specificity have been obtained for the following concentrations of Aβ peptide (pg/mL) with corresponding OR and confidence intervals (CI):
**Aβ₁₋₄₀=** 120.69. OR = 4.97 CI95% [1.64-15.04], p = 0.0045
**Aβ₁₋₄₂=** 39.75. OR = 2.88 CI95% [1.28-6.51], p = 0.01
**Aβₓ₋₄₀=** 172.25. OR = 2.83 CI95% [1.24-6.43], p = 0.013
**Aβₓ₋₄₂=** 33.00. OR = 2.25 CI95% [1.01-5.02], p = 0.05

Thus statistical analysis provides Aβ peptide concentrations in blood plasma, which may be used as reference values for determining the occurrence of brain dysfunction in individuals with DM1 disease.

### BIBLIOGRAPHY

Arold S, Sullivan P, Bilousova T, Teng E, Miller CA, Poon WW, Vinters HV, Cornwell LB, Saing T, Cole GM & Gylys KH (2011). Apolipoprotein E level and cholesterol are associated with reduced synaptic amyloid beta in Alzheimer's disease and apoE TR mouse cortex. Acta Neuropathol. 123: 39-52.
International Myotonic Dystrophy Consortium (IDMC) (2000) New nomenclature and DNA testing guidelines for myotonic dystrophy type 1 (DM1). Neurology 54: 1218-1221.
Bibl M, Welge V, Schmidt H, Esselmann H, Mollenhauer B, Lewczuk P, Otto M, Kornhuber J, Wiltfang J. (2012) Plasma amyloid-beta peptides in acute cerebral ischemia: a pilot study. J Clin Lab Anal. 26(4):238-45.
Bibl M, Welge V, Esselmann H & Wiltfang J (2012) Stability of amyloid-β peptides in plasma and serum. Electrophoresis 33: 445-450.
Cheng S, Barcelo JM, Korneluk RG (1996) Characterization of large CTG repeat expansions in myotonic dystrophy alleles using PCR, Hum Mutat 7(4): 304-310.
Dhaenens CM, Tran H, Frandemiche ML, Carpentier C, Schraen-Maschke S, Sistiaga A, Goicoechea M, Eddarkaoui S, Van Brussels E, Obriot H, Labudeck A, Gevaert MH, Fernandez-Gomez F, Charlet-Berguerand N, Deramecourt V, Maurage CA, Buee L, de Munain AL, Sablonniere B, Caillet-Boudin ML, Sergeant N (2011) Mis-splicing of Tau exon 10 in myotonic dystrophy type 1 is reproduced by overexpression of CELF2 but not by MBNL1 silencing. Biochim Biophys Acta 1812:732-742.
Gonzalez et al. (2000). New nomenclature and DNA testing guidelines for myotonic dystrophy type 1 (DM1). Neurology (0028-3878), 54 (6), p. 1218.
Hansson O, Stomrud E, Vanmechelen E, Östling S, Gustafson DR, Zetterberg H, Blennow K & Skoog I (2012) Evaluation of plasma Aβ as predictor of Alzheimer's disease in older individuals without dementia: a population-based study. J Alzheimers Dis 28: 231-238.
Head E, Doran E, Nistor M, Hill M, Schmitt FA, Haier RJ & Lott IT (2011) Plasma amyloid-β as a function of age, level of intellectual disability, and presence of dementia in Down syndrome. J Alzheimers Dis 23: 399-409.
Ho TH, Savkur RS, Poulos MG, Mancini MA, Swanson MS, Cooper TA (2005). Colocalization of muscleblind with RNA foci is separable from mis-regulation of alternative splicing in myotonic dystrophy. J Cell Sci 118: 2923-2933.
Huang Y, Potter R, Sigurdson W, Kasten T, Connors R, Morris JC, Benzinger T, Mintun M, Ashwood T, Ferm M, Budd SL, Bateman RJ. (2012) β-amyloid Dynamics in Human Plasma. Arch Neurol. 69(12):1591-7. doi: 10.1001/archneurol.2012.18107.
Itoh K, Mitani M, Kawamoto K, Futamura N, Funakawa I, Jinnai K, & Fushiki S (2010). Neuropathology does not Correlate with Regional Differences in the Extent of Expansion of CTG Repeats in the Brain with Myotonic Dystrophy Type 1. Acta Histochemica et Cytochemica, 43(6), 149-156.
Du H, Cline MS, Osborne RJ, Tuttle DL, Clark TA, Donohue JP, et al. (2010). Aberrant alternative splicing and extracellular matrix gene expression in mouse models of myotonic dystrophy. Nature Structural & Molecular Biology, 17(2), 187-193.
Jiang H, Mankodi A, Swanson MS, Moxley RT, Thornton CA (2004) Myotonic dystrophy type 1 is associated with nuclear foci of mutant RNA, sequestration of muscleblind proteins and deregulated alternative splicing in neurons. Hum Mol Genet 13: 3079-3088.
Kamsteeg EJ, Kress W, Catalli C, Hertz JM, Witsch-Baumgartner M, Buckley MF, van Engelen BG, Schwartz M, Scheffer H. (2012) Best practice guidelines and recommendations on the molecular diagnosis of myotonic dystrophy types 1 and 2. Eur J Hum Genet. 2012 Dec;20(12):1203-8.
Kiuchi A, Otsuka N, Namba Y, Nakano I, Tomonaga M. (1991) Presenile appearance of abundant Alzheimer's neurofibrillary tangles without senile plaques in the brain in myotonic dystrophy. Acta Neuropathol. 82(1):1-5).
Lachno, DR., Evert, BA., Vanderstichele, H., Robertson, M., Demattos, RB., Konrad, RJ., et al. (2013). Validation of assays for measurement of amyloid-β peptides in cerebrospinal fluid and plasma specimens from patients with Alzheimer's disease treated with solanezumab. Journal of Alzheimer's disease : JAD, 34(4), 897-910.
Lambert JC, Schraen-Maschke S, Richard F, Fiévet N, Rouaud O, Berr C, Dartigues J-F, Tzourio C, Alpérovitch A, Buee L & Amouyel P (2009) Association of plasma amyloid beta with risk of dementia: the prospective Three-City Study. Neurology 73: 847-853.
Lambert J-C, Dallongeville J, Ellis KA, Schraen-Maschke S, Lui J, Laws S, Dumont J, Richard F, Cottel D, Berr C, Ames D, Masters CL, Rowe CC, Szoeke C, Tzourio C, Dartigues J-F, Buée L, Martins R & Amouyel P (2011) Association of Plasma Aß Peptides with Blood Pressure in the Elderly. PLoS ONE 6: e18536.
Maruyama M, Shimada H, Suhara T, Shinotoh H, Ji B, Maeda J, Zhang MR, Trojanowski JQ, Lee VMY, Ono M, Masamoto K, Takano H, Sahara N, Iwata N, Okamura N, Furumoto S, Kudo Y, Chang K, Saido TC, Takashima A, Lewis J, Jang MK, Aoki I, Ito H and Higuchi M (2013) Imaging of Tau Pathology in a Tauopathy Mouse Model and in Alzheimer Patients Compared to Normal Controls, Neuron 79: 1094-1108.
Mattsson N, Andreasson U, Persson S, Carrillo MC, Collins S, Chalbot S, Cutler N, Dufour-Rainfray D, Fagan AM, Heegaard NHH, Hsiung G-YR, Hyman B, Iqbal K, Lachno DR, Lleó A, Lewczuk P, Molinuevo JL, Parchi P, Regeniter A, Rissman R, et al (2013) CSF biomarker variability in the Alzheimer's Association quality control program. Alzheimer's & Dementia 9: 251-261.
Maurage CA, Udd B, Ruchoux MM, Vermersch P, Kalimo H, Krahe R, Delacourte A, Sergeant N (2005) Similar brain tau pathology in DM2/PROMM and DM1/Steinert disease. Neurology 65: 1636-1638.
Meola G, Sansone V (2007) Cerebral involvement in myotonic dystrophies. Muscle Nerve 36: 294-306.
Metti AL, Cauley JA, Ayonayon HN, Harris TB, Rosano C, Williamson JD & Yaffe K (2012) The Demographic and Medical Correlates of Plasma Aβ40 and Aβ42. Alzheimer Disease & Associated Disorders.
Peric S, Mandic-Stojmenovic G, Markovic I, Stefanova E, Ilic V, Parojcic A, Misirlic-Dencic S, Ostojic M, Rakocevic-Stojanovic V, Kostic V. (2013) Cerebrospinal fluid biomarkers of neurodegeneration in patients with juvenile and classic myotonic dystrophy type 1. Eur J Neurol. July 3.
Perret-Liaudet A, Pelpel M, Tholance Y, Dumont B, Vanderstichele H, Zorzi W, Elmoualij B, Schraen S, Moreaud O, Gabelle A, Thouvenot E, Thomas-Anterion C, Touchon J, Krolak-Salmon P, Kovacs GG, Coudreuse A, Quadrio I, Lehmann S. Risk of Alzheimer's disease biological misdiagnosis linked to cerebrospinal collection tubes. J Alzheimers Dis. 2012;31(1):13-20.
Piccinni A, Veltri A, Vizzaccaro C, Catena Dell'Osso M, Medda P, Domenici L, Vanelli F, Cecchini M, Franceschini C, Conversano C, Marazziti D, Dell'Osso L. (2013) Plasma amyloid-β levels in drug-resistant bipolar depressed patients receiving electroconvulsive therapy. Neuropsychobiology.2013;67(4):185-91.
Potter R, Patterson BW, Elbert DL, Ovod V, Kasten T, Sigurdson W, Mawuenyega K, Blazey T, Goate A, Chott R, Yarasheski KE, Holtzman DM, Morris JC, Benzinger TLS & Bateman RJ (2013) Increased in Vivo Amyloid- 42 Production, Exchange, and Loss in Presenilin Mutation Carriers. Science Translational Medicine 5: 189ra77-189ra77.
Schupf N, Zigman WB, Tang MX, Pang D, Mayeux R, Mehta P, Silverman W. (2010) Change in plasma Aß peptides and onset of dementia in adults with Down syndrome. Neurology. 75(18):1639-44.
Sergeant N, Sablonniere B, Schraen-Maschke S, Ghestem A, Maurage CA, Wattez A, Vermersch P, Delacourte A (2001) Dysregulation of human brain microtubule-associated tau mRNA maturation in myotonic dystrophy type 1. Hum Mol Genet 10: 2143-2155.
Sergeant N, David JP, Champain D, Ghestem A, Wattez A, Delacourte A (2002) Progressive decrease of amyloid precursor protein carboxy terminal fragments (APP-CTFs), associated with tau pathology stages, in Alzheimer's disease. J Neurochem 81:663-672.
Sistiaga A, Urreta I, Jodar M, Cobo AM, Emparanza J, Otaegui D, Poza JJ, Merino JJ, Imaz H, Marti-Masso JF, Lopez de Munain A (2010) Cognitive/personality pattern and triplet expansion size in adult myotonic dystrophy type 1 (DM1): CTG repeats, cognition and personality in DM1. Psychol Med 40: 487-495.
Spies PE, Verbeek MM, van Groen T, Claassen JA. (2012) Reviewing reasons for the decreased CSF Abeta42 concentration in Alzheimer disease. Front Biosci. 17: 2024-34.
Suh YH, Checler F (2002) Amyloid precursor protein, presenilins, and alpha-synuclein: molecular pathogenesis and pharmacological applications in Alzheimer's disease. Pharmacol Rev. 54(3): 469-525.
Takami et al. (2009) gamma-Secretase: successive tripeptide and tetrapeptide release from the transmembrane domain of beta-carboxyl terminal fragment. The Journal of Neuroscience 29(41): 13042-13052.
Vermersch P, Sergeant N, Ruchoux MM, Hofmann-Radvanyi H, Wattez A, Petit H, Dwailly P, Delacourte A (1996) Specific tau variants in the brains of patients with myotonic dystrophy. Neurology 47: 711-717.
Vingtdeux V, Hamdane M, Loyens A, Gele P, Drobeck H, Begard S, Galas MC, Delacourte A, Beauvillain JC, Buee L, Sergeant N (2007) Alkalizing drugs induce accumulation of amyloid precursor protein by-products in luminal vesicles of multivesicular bodies. J Biol Chem 282:18197-18205.
Winblad S, Hellstrom P, Lindberg C, Hansen S (2006) Facial emotion recognition in myotonic dystrophy type 1 correlates with CTG repeat expansion. J Neurol Neurosurg Psychiatry 77: 219-223.
Winblad S, Mansson JE, Blennow K, Jensen C, Samuelsson L, Lindberg C (2008) Cerebrospinal fluid tau and amyloid beta42 protein in patients with myotonic dystrophy type 1. Eur J Neurol 15:947-952.
Yoshimura N, Otake M, Igarashi K, Matsunaga M, Takebe K, Kudo H. (1990) Topography of Alzheimer's neurofibrillary change distribution in myotonic dystrophy. Clin Neuropathol. 9(5):234-9.

### SEQUENCE LISTING

**SEQ ID NO 1**
**SEQ ID NO 2**
   DAEFRHDSGYEVHHQKLVFFAEDVGSNKGAIIGLMVGGVV
**SEQ** ID **NO 3**
   DAEFRHDSGYEVHHQKLVFFAEDVGSNKGAIIGLMVGGVVIA
**SEQ** ID **NO 4**
   CACAGGCTGAAGTGGCAGTTCCA
**SEQ ID NO 5**
   TGTCGGGGTCTCAGTGCATCCA

## Claims

1. An *in vitro* method for detecting the occurrence of Myotonic Dystrophy type 1 (DM1) disease in an individual, comprising the steps of:
a) providing a blood sample from said individual,
b) measuring an amount of Aβ peptide within said blood sample,
c) comparing the amount measured at step b) with a reference value,
d) determining the occurrence of DM1 in said individual from the comparison performed at step c).

2. The *in vitro* method according to claim 1, wherein the occurrence of DM1 disease in said individual is indicative of the occurrence of a brain dysfunction.

3. An *in vitro* method for discriminating between DM1 and a non-DM1 neurodegenerative disease in an individual, comprising the steps of:
a) providing a blood sample from said individual,
b) measuring an amount of Aβ peptide within said blood sample,
c) comparing the amount of Aβ peptide within said blood sample with a reference value,
d) discriminating between Myotonic Dystrophy type 1 (DM1) disease and a non-DM1 neurodegenerative disease from the comparison performed at step c).

4. The *in vitro* method according to claim 3, wherein the non-DM1 neurodegenerative disease is selected in a group comprising: Alzheimer's disease, frontolobar degeneration, Lewy body dementia, Parkinson's disease, Tauopathies and Synucleopathies.

5. An *in vitro* method for determining the efficacy of a treatment of Myotonic Dystrophy type 1 (DM1) disease in an individual, comprising the steps of:
a) providing a blood sample from said individual,
b) measuring an amount of Aβ peptide within said blood sample,
c) comparing the amount measured at step b) with a reference value,
d) determining the efficacy of said treatment in said individual from the comparison performed at step c).

6. The method according to any one of the preceding claims, wherein the blood sample is selected in a group comprising a plasma sample and a serum sample.

7. The method according to any one of the preceding claims, wherein the Aβ peptide is selected in a group comprising: Aβ_{Total}, Aβ₁₋₄₀, Aβ₁₋₄₂, Aβₓ₋₄₀ and Aβₓ₋₄₂, and wherein x ranges from 1 to 17.

8. The method according to any one of the preceding claims, further comprising a step of measuring the ratio between at least two different Aβ peptides selected in a group comprising: Aβ₁₋₄₀, Aβ₁₋₄₂, Aβₓ₋₄₀ and Aβₓ₋₄₂, and wherein x ranges from 1 to 17.

9. The method according to any one of the preceding claims, wherein the amount of Aβ peptide in the blood sample is measured by bringing into contact the sample with an Aβ peptide binding molecule.

10. The method according to claim 9, wherein the amount of Aβ peptide in the blood sample is measured by an ELISA method.

11. The method according to any one of the preceding claims, further comprising a step of measuring an amount of Aβ peptide within a cerebrospinal fluid (CSF) sample from said individual.

12. The method according to claim 11, comprising a step of comparing the amount of Aβ peptide within said CSF sample to a second reference value.

13. The method according to any one of the preceding claims, further comprising a step of measuring the number of CTG repeats on at least one DMPK allele from said individual.

14. The method according to any one of the preceding claims, wherein the individual's genomic DNA comprises at least one expanded DMPK allele of at least 50 CTG repeats.

15. An assay for the screening of a candidate agent potentially useful in the treatment of DM1 in an individual, said assay comprising determining whether a tested agent can modulate, such as increase or reduce and preferably reduce, the level of an Aβ peptide in a blood sample from said individual.
